(19) Europäisches Patentamt
European Patent Office
Office européen des brevets

(11) Veröffentlichungsnummer: **0 140 084**
**B1**

(12) # EUROPÄISCHE PATENTSCHRIFT

(45) Veröffentlichungstag der Patentschrift: **13.06.90**

(51) Int. Cl.$^5$: **C 07 K 7/40**, C 12 P 21/02, A 61 K 37/26

(21) Anmeldenummer: **84110813.7**

(22) Anmeldetag: **11.09.84**

(54) **Verfahren zur Herstellung von Insulin-Derivaten, deren B-Kette C-terminal verlängert ist, neue basisch modifizierte Insulin-Derivate, diese enthaltende Mittel und ihre Verwendung.**

(30) Priorität: **17.09.83 DE 3333640**

(43) Veröffentlichungstag der Anmeldung: **08.05.85 Patentblatt 85/19**

(45) Bekanntmachung des Hinweises auf die Patenterteilung: **13.06.90 Patentblatt 90/24**

(84) Benannte Vertragsstaaten: **AT BE CH DE FR GB IT LI LU NL SE**

(56) Entgegenhaltungen:
**EP-A-0 017 938**
**EP-A-0 045 187**
**EP-A-0 056 951**
**EP-A-0 085 516**
**EP-A-0 089 007**
**EP-A-0 092 280**

**Die Akte enthält technische Angaben, die nach dem Eingang der Anmeldung eingereicht wurden und die nicht in dieser Patentschrift enthalten sind.**

(73) Patentinhaber: **HOECHST AKTIENGESELLSCHAFT**
**Postfach 80 03 20**
**D-6230 Frankfurt am Main 80 (DE)**

(72) Erfinder: **Obermeier, Rainer, Dr.**
**Langenhainer Weg 14**
**D-6234 Hatterheim am Main (DE)**
Erfinder: **Geiger, Rolf, Prof.Dr.**
**Heinrich-Bleicher-Strasse 33**
**D-6000 Frankfurt am Main 50 (DE)**
Erfinder: **Grau, Ulrich, Dr.**
**Zeil 17**
**D-6238 Hofheim am Taunus (DE)**

(56) References cited:
**Excerpta Medica International Congress Series No. 231 R.E. Chance "Chemical, physical, biological and immunological studies on poreine proinsulin and related polypeptides" Seiten 292-305**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**EP 0 140 084 B1**

Courier Press, Leamington Spa, England.

**Beschreibung**

Insulin-Arg$^{B31}$-OH, Insulin-Arg$^{B31}$-Arg-$^{B32}$-OH und anderc in der B-Kette C-terminal basisch modifizierte Insuline weisen in Arzneimitteln, ohne spezielle Zusätze wie surfen$^{R}$ oder Protamin Depotcharakter auf. Solche Insuline, Verfahren zu ihrer Herstellung, diese enthaltende Mittel und ihre Verwendung sind bereits vorgeschlagen worden (deutsche Patentanmeldungen P 33 26 472.4, P 33 26 473.2, P 33 27 709.5 und P 33 27 928.4). Die Vorteile solcher verzögert wirkender Insulin-Zubereitungen sind in der basseren Verträglichkeit und, bei der mit der Insulinbehandlung verbundenen Langzeitbehandlung, vor allem in der fehlenden Immunogenität des Depothilfsmittel zu finden.

Bei der Biosynthese von Insulin entsteht aus dem unmittelbaren Vorläufer, dem einkettigen Proinsulin, durch enzymatische Abspaltung des sog. Connecting-Peptids das zweikettige Insulin. Durch unvollständige Processing sind aber auch sog. Intermediat-Insuline in sehr geringen Mengen im isolierten Rohinsulin zu finden. Diese Zwischenprodukte der Insulinbiosynthese sind strukturell aufgeklärt. Sie bestehen überwiegend aus B31-Arg- und B31-32-Arg-Arg-Insulinderivaten.

Behandelt man in vitro Proinsuline von Rind, Schwein oder Mensch mit Trypsin (Chance, Excerpta Medica International Congress Series No. 231, Seite 292 ff), so entsteht neben den entsprechenden Desoktapeptid-B23-30-Insulinen vor allem ein Gemisch aus B31-Arg- und B32-Arg-Arg-Insulinen. Die sichere Trennung und Hochreinigung dieser Produkte ist sehr aufwendig und erfordert mehremalige Ionenaustauscherchromatographie, die mit Verlusten verbunden ist. Die Gewinnung präparativer Mengen aus Rohinsulin zur Anwendung in der Therapie ist daher nicht praktikabel. Außerdem stehen die dem Humaninsulin entsprechenden Produkte nicht zur erfügung.

Ein möglicher Weg zur Gewinnung solcher Derivate besteht darin, gentechnologisch hergestelltes Proinsulin, oder Analoga davon, enzymatisch zu spalten. Wie erwähnt, entstehen durch das Reinigungsverfahren Verluste.

Aufgabe der vorliegenden Erfindung ist es, ein Verfahren zu finden, mit dem die für eine therapeutische Anwendung der neuartigen galenischen Zubereitungen benötigten Mengen an basisch modifizierten Insulin-Derivaten auf einfache Weise hergestellt werden können.

Das im folgenden beschriebene erfindungsgemäße Verfahren löst die Aufgabe durch enzymkatalysierte Umwandlung von billigem biosynthetischem Insulin, wie Humaninsulin oder Schweineinsulin unter Verwendung der geeigneten Peptid zu den gewünschten Produkten.

In den letzten Jahren sind verschiedene chemische und enzymatische Verfahren bekannt geworden (Biochem, J. *211* (1983) 671—676, US-Patente 3 903 068, 3 276 961, 4 320 197, GB—A—2069502, u.a.), mit deren Hilfe man z.B. Schweineinsulin umwandeln kann. Eine solches ist z.B. auch das in der EP—A—56951 beschriebene Verfahren, gemäß welchem man Humaninsulin oder seine Derivate aus Schweineinsulin bzw. aus Schweineinsulin-Derivaten durch Umsetzung mit einem Überschuß eines Threoninesters in Gegenwart von Trypsin oder einem Trypsin-ähnlichen Ferment bei bestimmten pH-Werten und gegebenenfalls nachfolgender Esterspaltung herstellen kann; Trypsin und Trypsin-ähnliche Fermente sind sogenannte Endopeptidasen, welche innerhalb von Peptidketten am Carboxylende von basischen Aminosäureresten angreifen.

EPO—A—00 85 516 und EP—A—00 45 187 beziehen sich auf ähnliche Verfahren zum Austausch der B30-Aminosäure von Insulinen durch eine andere Aminosäure oder eines von dessen Derivaten, in erster Linie zum Zwecke der Herstellung von Humaninsulin aus Schweineinsulin. Bei diesen Verfahren wird die B30-Aminosäure des jeweiligen Ausgangs-Insulins enzymatisch durch eine andere Aminosäure oder eines von dessen Derivaten ausgetauscht. Die für diese Austauschreaktion (Transpeptidierung) verwendeten Enzyme (wie das hier besonders bevorzugte Enzym Carboxypeptidase Y) sind sog. Exopeptidasen, welche Peptidketten vom C-terminalen Ende her abbauen. Wenn für die Transpeptidierung Aminosäure-Derivate eingesetzt werden, müssen die Produkte am Ende noch ent-derivatisiert werden.

Darüber hinaus sind auch Verfahren vorgeschlagen worden, die die Umwandlung von geeigneten Präproinsulinderivaten und Intermediat-Insulinen z.B. zu Humaninsulin erlauben (deutsche Patentanmeldung P 32 09 184.2)

Die Erfindung betrifft ein Verfahren zur Herstellung eines Insulin-Derivats der Formel I,

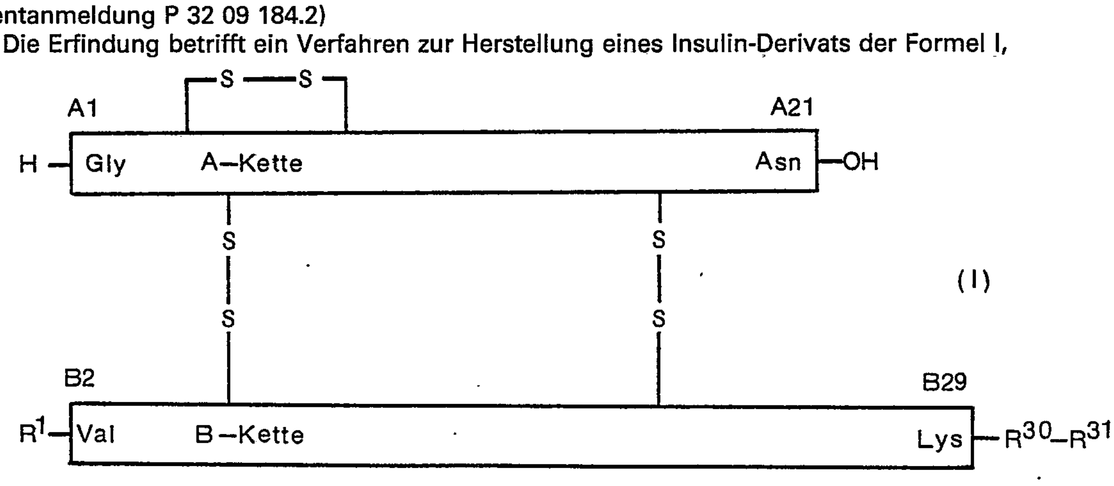

(I)

in welcher

$R^1$ H oder H-Phe bedeutet,

a) $R^{30}$ für den Rest einer neutralen, natürlich vorkommenden L-Aminosäure und

$R^{31}$ für eine physiologisch unbedenkliche organische Gruppe basischen Charakters steht, bestehend aus bis zu 3 α-Aminosäuren, deren endständige Carboxyfunktion frei ist, oder

b) $R^{30}$ für den Rest einer basischen, natürlich vorkommenden L-Aminosäure und

$R^{31}$ für eine physiologisch unbedenkliche organische Gruppe neutralen oder basischen Charakters steht, bestehend aus bis zu 3 α-Aminosäuren, deren endständige Carboxyfunktion frei ist, und welches einen isoelektrischen Punkt über 5,8 aufweist, das dadurch gekennzeichnet ist, daß man ein Insulin der Formel I, in welcher

$R^1$ H oder H-Phe bedeutet,

$R^{30}$ den Rest einer genetisch kodierbaren L-Aminosäure bedeutet und

$R^{31}$ für OH oder eine Schutzgruppe der Carboxy Funktion steht, mit einem Peptid der Formel III

$$H—R^{30}—R^{31} \qquad (III),$$

in welcher

a) $R^{30}$ für den Rest einer neutralen, natürlich vorkommenden L-Aminosäure und

$R^{31}$ für eine physiologisch unbedenkliche organische Gruppe basischen Charakters steht, bestehend aus bis zu 3 α-Aminosäuren, deren endständige Carboxyfunktion frei ist, oder

b) $R^{30}$ für den Rest einer basischen, natürlich vorkommenden L-Amnosäure und

$R^{31}$ für eine physiologisch unbedenkliche organische Gruppe neutralen oder basischen Charakters steht, bestehend aus bis zu 3 α-Aminosäuren, deren endständige Carboxyfunktion frei ist, in Gegenwart einer trypsinähnlichen Endopeptidase in Wasser, gegebenenfalls unter Zusatz eines geeigneten organischen Lösungsmittels bei einem pH-Wert unterhalb des isoelektrischen Punktes der Insuline, vorzugsweise unterhalb pH 5,4 umsetzt und gegebenenfalls in den Seitenketten eingeführte Schutzgruppen abspaltet.

Überraschenderweise gelingt die Transpeptididierung der Ausgangs-Insuline mit den Peptiden der Formel III (mit endständiger freier Carboxyfunktion!) in Kommerziell ausreichenden Maß nur Gegenwart von trypsinähnlichen Endspeptidagen als Enzymen, nichts dagegen in Gegenwart von Trypsin.

Bevorzugt werden solche Verbindungen der Formel I hergestellt, in wlecher $R^1$ für h-Phe steht und/oder $R^{30}$ Ala, Thr oder Ser bedeutet. Weiterhin stellt man vorzugsweise Verbindungen her, in welchen die A-Kette und die Kette (B2—30) die Sequenz des Humaninsulins aufweisen.

Nach dem erfindungsgemäßen Verfahren lassen sich beispielsweise herstellen:

Des-$Phe^{B1}$-Schweineinsulin-$Arg^{B31}$-OH
Des-$Phe^{B1}$-Humaninsulin-$Arg^{B31}$-OH
Des-$Phe^{B1}$-Schweineinsulin-$Arg^{B31}$-$Arg^{B32}$-OH
Des-$Phe^{B1}$-Humaninsulin-$Arg^{B31}$-$Arg^{B32}$-OH
Des-$Thr^{B30}$-Humaninsulin-$Val^{B30}$-$Ala^{B31}$-$Arg^{B32}$-OH
Humaninsulin-$Lys^{B31}$-OH
Humaninsulin-D-$Arg^{B31}$-OH
Humaninsulin-D-$Arg^{B31}$-$Arg^{B32}$-OH
Humaninsulin-$Arg^{B31}$-D-$Arg^{B32}$-OH
Humaninsulin-$Lys^{B31}$-$Arg^{B32}$-OH
Humaninsulin-$Arg^{B31}$-$Lys^{B32}$-OH
Humaninsulin-$Val^{B31}$-$Arg^{B32}$-OH
Humaninsulin-$Val^{B31}$-$Arg^{B31}$-$Arg^{B33}$-OH
Humaninsulin-$Lys^{B31}$-$Arg^{B32}$-$Arg^{B33}$-OH
Humaninsulin-$Orn^{B31}$-OH
Humaninsulin-$Leu^{B31}$-$Cit^{B32}$-OH
Des-$Thr^{B30}$-Humaninsulin-$Arg^{B30}$-$Arg^{B31}$-OH
Des-$Thr^{B30}$-Humaninsulin-$Lys^{B30}$-$Ala^{B31}$-$Arg^{B32}$-OH
insbesondere aber
Humaninsulin-$Arg^{B31}$-OH
Humaninsulin-$Arg^{B31}$-$Arg^{B32}$-OH

Genetisch kodierbar sind die folgenden L-Aminosäuren: *Gly, Ala, Ser, Thr, Val, Leu, Ile,* Asp *Asn, Glu, Gln, Cys, Met,* Arg, Lys, His, *Tyr, Phe,* Trp, *Pro* (neutrale Aminosäuren unterstrichen).

Unter einer neutralen, natürlich vorkommenden Aminosäuren versteht man insbesondere Gly, Ala, Ser, Thr, Val, Leu, IIle, Asn, Gln, Cys, Met, Tyr, Phe, Pro oder Hyp. Unter einer basischen, natürlich vorkommenden Aminosäure versteht man insbensondere Arg, Lys, Hyl, Orn, Cit oder His.

Als Insuline können alle natürlich isolierten Insuline mit Lys (B29), bevorzugt jedoch diejenigen vom Schwein und Mensch eingesetzt werden.

Des-$Phe^{B1}$-Insuline als Ausgangsverbindungen für das erfindungsgemäße Verfahren sind beispielsweise aus der DE—PS 20 05 658 oder aus der EP—A—46 979 bekannt.

Als Peptide oder Aminosäurederivate, die zur enzymkatalysierten Reaktion eingesetzt werden können, kommen vor allem gegebenenfalls in der Seitenkette, ober nicht am Carboxylende geschützte Di-, Tri-, und Tetrapeptide mit Gly, L- oder D-Aminosäuren als N-Terminus in Frage.

Urethanschutzgruppen der ε-Aminofunktion von Lys oder Orn sind beispielsweise Fmoc, Fcboc, Z, Boc, Ddz, Bpoc, Z ($NO_2$), Pyoc, Dobz, Moc, Mboc, Iboc, Adoc, Adpoc, Msc oder Pioc; bevorzugt sind Z oder Boc. Die Entfernung dieser Aminoschutzgruppen erfolgt mit Säuren, Basen oder reduktiv (vgl. Kontakte Merck 3/79, S. 14 ff.).

Schutzgruppen der Guanidinogruppe des Arginins sind z.B. Adoc, Tosyl, Boc, Z, Mesitylen-2-sulfonyl (Mts) u.ä Die Abspaltung kann hydrolytisch oder hydrogenolytisch erfolgen (vgl. Kontakte Merck 1/80, S. 23,30).

Die OH-Seitenfunktion von Thr oder Ser kann in bekannter Weise durch Ether- oder Acylschutzgruppen wie z.B. ($C_1$ bis $C_6$)-Alkyl, vorzugswiese tert. Butyl oder ($C_1$ bis $C_6$)-Alkanoyl blockiert werden.

Die Auswahl der Schutzgruppen und die Synthesestrategie wird von der Art der Aminosäuren bestimmt.

Als bevorzugte Dipeptide können solche verwendet werden, bei denen ein N-terminaler Alanyl- oder Threonylrest der mit einem L-/D-Arginin- oder L-/D-Lysinrest verknüpft ist.

Die obengenannten Peptide werden nach Standardverfahren synthetisiert, wie sie beispielsweise in "The Peptides Analysis, Synthesis, Biology, Vol. 1 Major Methods of Peptide Bond Formation, Part A", ed. E. Gross, J. Meierhofer, Academic Press N.Y. (1979) beschrieben sind.

Für das erfindungsgemäße Verfahren eignen sich solche Endopeptidasen, die aus der Literatur als trypsinähnlich bekannt sind, d.h. solche die spezifisch Peptidbindungen am Carboxyende basischer Aminosäuren spalten (vgl. EP—A—17938).

Die zur enzymatischen Umsetzung eingesetzten Peptide werden vorzugsweise im 40—150 fach molaren Überschuß, bezogen auf die Insulinkomponente verwendet. Als Enzym-Substrat-Verhältnis kann ein Gewichtsverhältnis von vorzugsweise 1:5 bis 1:20, insbesondere 1:10, von Enzym zur Insulinkomponente verwendet werden.

Um Peptide, die geringere Wasserlöslichkeit zeigen, zur Reaktion zu bringen, kann for ansonsten wäßrigen Reaktionslösung ein geeignets, mit Wasser mischbares organisches Lösungsmittel wie Methanol, Dimethylformamid oder Dioxan bis zur homogenen Lösung zugegeben werden.

Der Wassergehalt des Reaktionsgemisches sollte jedoch nicht unter 50% des Gemisches aus Wasser/organischem Lösungsmittel sinken, damit ausbeutemindernde Denaturierung von Insulinkomponete und/oder des Enzyms unterdrückt werden.

Die pH-Werte bei welchen die enzymatische Reaktion durchgeführt wird, liegen gewöhnlich zwischen 4—5 vorzugsweise bei etwa pH 4,5. Bei höheren pH-Werten werden die Ausbeuten der bevorzugten Transamidierungsreaktionen an der Position $Lys^{B29}$ durch unerwünschte proteolytische Spaltung am $Arg^{B22}$ reduziert.

Allen nach dem Erfindungsgemäßen Verfahren erhaltenen bzw. erhältlichen Insulinderivaten ist gemeinsam, daß die zusätzliche(n), an der Oberfläche des Moleküls befindliche(n) positive(n) Ladung(en) dem Molekül inen in den Neutralbereich hinein verschoben isoelektrischen Punkt verleihen. Je nach Derivat werden isoelektrische Punkte von 5,8 bis etwa 8,5, insbesondere 6,2 bis 8,2 in der isoelektrischen Fokussierung gemessen. Damit sind die Derivate im Neutralbereich weniger löslich als natives Insulin oder Proinsulin, die ihren isoelektrischen Punkt und damit den Bereich maximaler Unlöslichkeit bei pH = 5,4 haben, während sie im Neutralbereich normaler weise gelöst vorliegen.

Die Löslichkeitseigenschaften von Insulin und Proinsulin lassen sich im Bereich oberhalb des isoelektrischen Punktes, d.h. im therapeutisch besonders interessanten Neutralbereich, durch Zugabe von Zinkionen beeinflussen. Zink wirkt dabei als Depotprinzip in der Weise, daß es den hexameren Zustand des Insulins sowie dessen Kristallisierungstendenz stabilisiert. Im subkutanen Gewebe lösen sich diese Aggregate wieder.

Ein weiteres geläufiges Depotprinzip ist die Kristallisation des Insulins oder Proinsulins als Komplex mit einem basischen Protein, z.B. Globin oder Protamin.

Bei der Anwendung des Proinsulins in Lösung oder in Verbindung mit einem der beschriebenen Depotprinzipien bedarf es eines weiteren proteolytischen Abbaus, um natives, voll wirksames Insulin freizusetzen. Intakes Proinsulin hat nur etwa 1/8 der biologischen Wirksamkeit des Insulins, weil, so die Vorstellung, ein Teil der biologisch aktiven Oberflächenregion, die Rezeptor-Bindungsregion, durch das im Proinsulin vorhandene C-Peptid maksiert ist. Als Proinsulin kommt allerdings für die Diabetestherapie nur homologes, also nur solches mit menschlicher Sequenz, in Frage (vgl. z.B. DE—AI—32 32 036). Heterologes Proinsulin besitzt eine signifikante Immunogenität. Es ist in diesem Zusammenhang bemerkenswert, daß auch humane Proinsuline Variationen im C-Peptidtel aufweisen können.

Insulin-$Arg^{B30}$-OH und andere Insulin-Derivate, deren B-Kette C-terminal eine organische Gruppe basischen Charakters trägt weisen, im Unterschied zum Proinsulin, eine etwa gleich hohe biologische Aktivität wie natives Insulin auf.

Die nach dem erfindungsgemäßen Verfahren erhaltenen bzw. erhältlichen basisch modifizierten Insulin-Derivate eignen sich zur Verwnedung in Arzneimitteln zur Behandlung des Diabetes mellitus.

Die Arzneimittel stellen neue Verzögerungsprinzipien dar, die ohne Dept-Hilfsstoffe, wie Zink oder Protaminsulfat, zur Wirung gebracht werden können. Die Depotwirkung geht auf ein inhärentes,

proteinchemisch bedingtes, physikalisches Prinzip, die Schwerlöslichkeit des Insulin-Derivats an dessen isoelektrischem Punkt, zurück. Seine Wiederauflösung unter physiologischen Bedingungen wird möglicherweise durch Abspaltung der zusätzlichen basischen Gruppen erreicht, die je nach Derivat durch tryptische oder trypsinähnlihe und/oder Carboxypeptidase B oder der Carboxypeptidase B ähnliche und/ oder Esterase-Aktivität zustante kommt. Die jeweils abgespaltenen Gruppen sind entweder rein physiologische Metaboliten, wie Aminosäuren, oder aber leicht metabolisierbare, physiologisch unbedenkliche Substanzen.

Die Insulin-Derivate sind auch im Unterschied zu in der Literatur beschriebenen Intermediaten, die noch Teile des heterologen C-Peptids enthalten, in der Tegel nicht stärker imunogen als das entsprechende Insulin selbst.

Die Arzneimittel enthalten als Wirkstoff eines oder mehrerer der neuen Insulin-Derivate der Formel I. Sie wiesen vorzugsweise einen pH-Wert zwischen 2,5 und 8,5 auf, enthalten eine geeignetes Isotoniemittel, ein geeignetes Konservierungsmittel und gegebenenfalls einen geeigneten Puffer für einen pH-Bereich zwischen 5,0 und 8,5.

Eine typische Anwendungsform der beschriebenen Derivate stellen Präparationen dar, die unterhalb des isoelektrischen Punktes als Lösungen in einem physiologisch unbedenklichen Träger vorliegen. Dabei kann der pH der Lösung typischerweise pH = 5,0 betragen, liegt also deutlich höher als der von sauren Altinsulinen (typischerweise pH = 3). Eine neutralere Injektionslösung bietet u.U. deutliche Vorteile in Bezug auf deren Verträglichkeit.

Eine weitere typische Anwendungsform sind Suspensionen aus amorphen oder kristallinen Niederschlägen der beschriebenen Derivate in einem physiologisch unbedenklichen Träger von etwa neutralem pH.

Es ist aber auch möglich, die in den Derivaten inhärente Schwelöslichkeit im physiologischen pH-Bereich durch zusätliche Depotprinzipien, wie etwa durch Zugabe von Zink oder Protaminsulfat, zu verstärken. Die zugesetzte Zinkmenge kann dabei bis zu 100 µg $Zn^{2+}$/100 Insulineinheiten, typischerweise etwa 50 µg $Zn^{2+}$/100 Insulineinheiten, betragen. Die Protaminmenge kann zwischen 0,28 mg und 0,6 mg pro 100 Einheiten betragen (bezogen auf Protaminsulfat). Auf diese Weise sind besonders lang wirksame Präparationen herstellbar, für die es in Zukunft breitere Andwendung als bisher geben wird, nachdem gerade eine Basalmenge an Insulin therapeutisch vorteilhaft erscheint. Dies ist schon jetzt eine Erkenntnis aus der Therapie mit Insulindosiergeräten.

Als physiologisch unbedenkliches und mit dem Insulinderivat verträgliches Trägermedium eignet sich eine sterile wäßrige Lösung, die zu Blut in der üblichen Weise, z.B. durch Glycerin, Kochsalz, Glucose, isotonisch gemacht wird und daneben noch eines der gebräuchlichen Konservierungsmittel z.B. Phenol, m-Kresol oder p-Hydroxybenzoesäureester, enthält. Das Trägermedium kann zusätzlich eine Puffersubstanz, z.B. Natriumacetat, Natriumcitrat, Natriumphosphat, enthalten. Zur Einstellung des pH werden verdünnte Säuren (typischerweise HCl) bzw. Laugen (typischerweise NaOH) verwendet.

Die Insulin-Derivate können in den Arnneimitteln auch als Alkali- oder Ammoniumsalze eingesetzt werden. Ein beliebiger Anteil eines oder mehrerer Insulin-Derivate der Formel I oder ein Insulin-Derivat der Formel I kann in einer Mischung weiterer dieser Insulin-Derivate unabhängig voneinander jeweils in gelöster, amorpher und/oder kristalliner Form vorliegen.

Es ist manchmal vorteilhaft, der entsprechenden Zubereitung eine geeignete Menge eines geeigneten Stabilisators zuzusetzen, der die Präzipitation von Protein bei thermischmechanischer Belastung bei Kontakt mit verschiedenen Materialien verhindert. Solche Stabilisatoren sind beispielsweise aus der EP—A—18609 der D—A—32 40 117 oder aus der WO—83/00288 bekannt.

Bei den Arnneimitteln, die auch eines der bekannten Verzögerungsprinzipien, wie etwa Protaminsulfat, Globin oder Zink in geeeigneten Mengen enthalten können , kann ein solches Verzögerungsprinzip in Kombination mit dem gesamten Wirkstoffanteil oder mit Teilen davon oder einem oder mehreren Insulin-Derivaten der Formel I in einer Mischung angewant werden. Ein Mittelkann verschiedene Insulin-Derivate der Formel I in Kombination mit mehreren verschiedenen verzögernd wirkenden Hilfsstoffen enthalten.

Mit den basisch modifizierten Insulin-Derivaten der Formel I sind vielfältige und sehr fein abstimmbare Wirkungscharakteristiken erzielbar; damit sollten nach den eingangs gemachten Bemerkungen Fortschritte insbeondere im Hinblick auf diabetische Spätkomplikationen verbunden sein. Das erfindundungsgemäße Verfahren kann analog wie in den nachfolgenden Vergleichsbeispielen (die nicht unter die Ansprüche fallen) durchgeführt werden:

Beispiele:

1) 6 g Insulin vom Schwein werden in 20 ml Wasser und 5 ml Eisessig zusammen mit 37 g H-Thr-Arg$(Adoc)_2$-OBut·Hac gelöst und mit Eisessig ein pH-Wert von 4,6 eingestellt. Nach Kühlen auf 0°C wird zur Lösung 0,6 g Trypsin gelöst in 2 ml Wasser gegeben. Die Reaktion wird 24 Stunden bei 4—10°C gehalten. Danach wird durch Zugabe von 200 ml Methanol oder Aceton das Reaktionsprodukt Humaninsulin-B31-Arg$(Adoc)_2$-OBut gefällt und abzentrifugiert. Der klare Überstand wird zur Zurückgewinnung des Dipeptidderivates aufgearbeitet.

Der Niederschlag wird noch einmal mit einem Gemisch aus Methanol/Ether gewaschen und im Vakuum getrocknet. Mit Hilfe der HPLC werden 47% an Produkt im Rohgemisch gemessen. Die Trennung und Reinigung HI-$Arg^{B31}(Adoc)_2$-OBut erfolgt an Kieselgel wie in der EP—A—82359 beschrieben.

HI-Arg$^{B31}$OH wird durch einstüngige Behandlung mit Trifluoressigsäure und anschließende Fällung mit Ether erhalten.

2) 600 mg Schweineinsulin werden in 2 ml $H_2O$, 0,8 ml DMF und 0,5 ml Essigsäure zusammen mit 12,6 g Hac·Thr·Arg(Adoc)$_2$-Arg(Adoc)$_2$·OBut gelöst und auf 0°C gekühlt. Dem Gemisch werden 60 mg Trypsin gelöst in 0,2 ml Wasser zugefügt. Nach 24 Stunden können in einer Probe mit Hilfe der HPLC 40% von HI-Arg$^{B31}$-(Adoc)$_2$-Arg$^{B32}$(Adoc)$_2$-OBut bestimmt werden.

3) 60 mg Schweineinsulin werden in 0,5 ml Wasser, 0,2 ml Dimethylformamid und 0,1 ml Essigsäure zusammen mit 676 mg Hac·2 HCl+Thr(But)-D-Arg-Arg(Adoc)$_2$-OBut gelöst. Der pH wird mit Triethylamin auf 5,0 gestellt und 6 mg Trypsin, gelöst in 0,1 ml Wasser, zugegeben. Nach 24 Stunden können mit Hilfe der HPLC 34% von Reaktionsprodukt HI-(B30)-(But)-D-Arg$^{B31}$-Arg$^{B32}$-(Adoc)$_2$-OBut bestimmt werden.

4) 6 g Humaninsulin werden in 20 ml Wasser und 5 ml Essigsäure zusammen mit 48 g HCl·Hae·Thr(But)·Arg(Adoc)$_2$-OBut gelöst und wie in Beispiel 1 mit 0,6 g Trypsin umgesetzt. Umsetzungerate nach 16 Stunden: 35%.

5) 85 mg Proinsulin von Schwein werden mit 483 mg Hac·HCl·Thr(But)-Arg(Adoc)$_2$-OBut in 0,5 ml $H_2O$ und 0,3 ml Essigsäure gelöst. Der pH-Wert wird auf 5,0 eingestellt und mg Trypsin gelöst in 0,1 ml Wasser zugegeben. Umsetzungsrate nach 24 h: 37%.

6) 85 mg Proinsulin vom Schwein werden mit 1,2 g Hac·Thr-Arg(Adoc)$_2$-Arg(Adoc)$_2$-OBut wie in Beispiel 5 umgesetzt: Umsetzungsrate mittels HPLC: 33%.

**Patentansprüche**

1. Verfahren zur Herstellung eines Insulin-Derivats der Formel I,

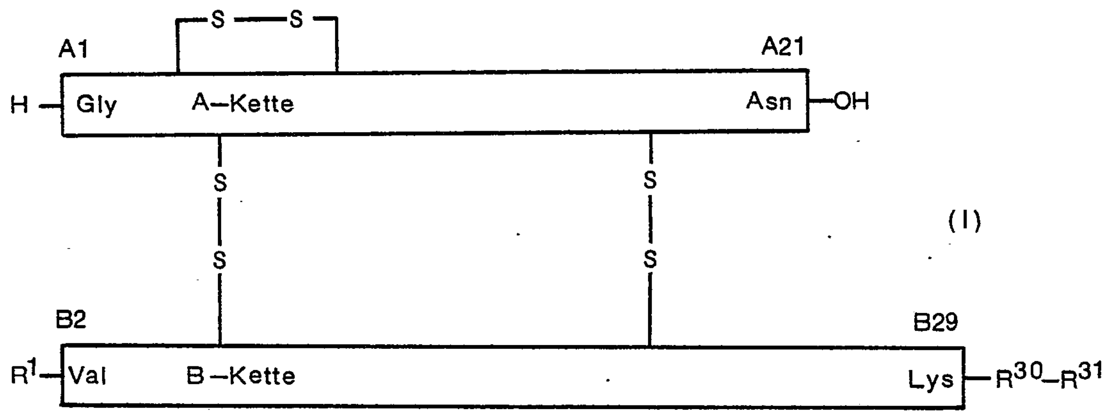

(I)

in welcher

$R^1$ H oder H-Phe bedeutet,

a) $R^{30}$ für den Rest einer neutralen, natürlich vorkommenden L-Amnosäure steht und

$R^{31}$ für eine physiologisch unbedenkliche organische Gruppe basischen Charakters steht, bestehend aus bis zu 3 α-Aminosäuren, deren endständige Carboxyfunktion frei ist, oder

b) $R^{30}$ für den Rest einer basischen, natürlich vorkommenden L-Amnosäure und

$R^{31}$ für eine physiologisch unbedenkliche organische Gruppe neutralen oder basischen Charakters steht, bestehend aus bis zu 3 α-Aminosäuren, deren endständige Carboxyfunktion frei ist, und welches einen isoelektrischen Punkt über 5,8 aufweist, das dadurch gekennzeichnet, daß man ein Insulin der Formel I, in welcher

$R^1$ H oder H-Phe bedeutet,

$R^{30}$ den Test einer genetisch kodierbaren L-Amnosäure bedeutet und

$R^{31}$ für OH oder eine Schutzgruppe der Carboxyfunktion steht, mit einem Peptid der Formel III

$$H—R^{30}—R^{31} \quad (III),$$

in welcher

a) $R^{30}$ für den Rest einer neutralen, natürlich vorkommenden L-Amnosäure und

$R^{31}$ für eine physiologisch unbedenkliche organische Gruppe basischen Charakters steht, bestehend aus bis zu 3 α-Aminosäuren, deren endständige Carboxyfunktion frei ist, oder

b) $R^{30}$ für den Rest einer basischen, natürlich vorkommenden L-Amnosäure und

$R^{31}$ für eine physiologisch unbedenkliche organische Gruppe neutralen oder basischen Charakters steht, bestehend aus bis zu 3 α-Aminosäuren, deren endständige Carboxyfunktion frei ist, in Gegenwart einer trypsinähnlichen Endopeptidase in Wasser, gegebenenfalls unter Zusatz eines geeigneten organischen Lösungsmittels bei einem pH-Wert unterhalb des isoelektrischen Punktes der Insuline umsetzt gegebenenfalls in den Seitenketten eingeführte Schutzgruppen abspaltet.

2. Verfahren nach Anspruch 1, dadurch gekennzeichnet, daß Insulin-Derivate der Formel I hergestellt werden, in welcher $R^1$ für H-Phe steht.

3. Verfahren nach einem der Ansprüche 1 oder 2, dadurch gekennzeichnet, daß Insulin-Derivate der Formel I hergestellt werden, in welcher als Rest $R^{30}$ für eine neutrale naturlich vorkommende L-Aminosäure Ala, Thr, oder Ser steht.

4. Verfahren nach einem oder mehreren der Ansprüche 1 bis 3, dadurch gekennzeichnet, daß Insulin-Derivate der Formel I hergestellt werden, in welcher die A-Kette und die Kette (B2—30) die Sequenz des Humaninsulins aufweisen.

5. Verfahren nach einem oder mehreren der Ansprüche 1 bis 4, dadurch gekennzeichnet, daß Humaninsulin-Arg$^{B31}$-OH oder Humaninsulin-Arg$^{B31}$-Arg$^{B32}$-OH hergestellt wird.

**Revendications**

1. Procédé pour la préparation d'un dérivé d'insuline de formule 1

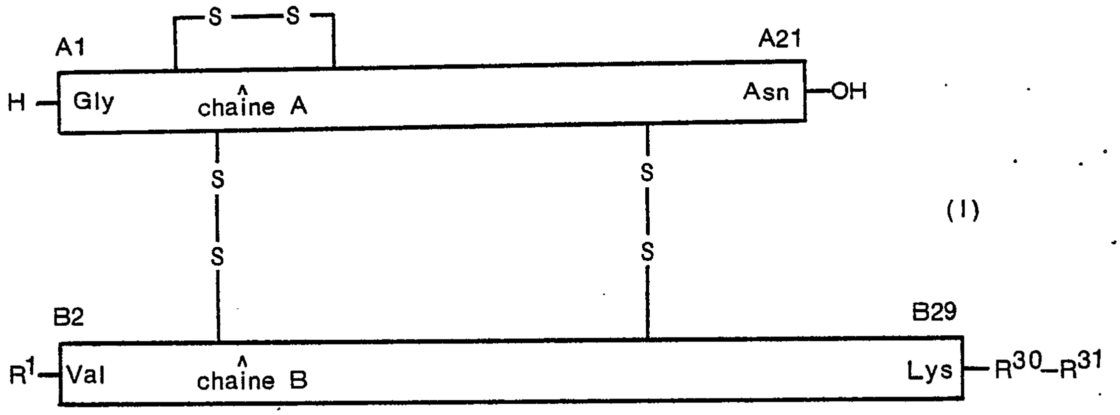

(I)

dans laquelle

$R^1$ représente H ou H-Phe,

a) $R^{30}$ représente le reste d'un L-aminoacide neutre existant dans la nature, et

$R^{31}$ représente un groupe organique physiologiquement acceptable, de caractère basique, constitué de jusqu'à 3 α-aminoacides, dont la fonction carboxy terminale est libre ou,

b) $R^{30}$ représente le reste d'un L-aminoacide basique, existant dans la nature, et

$R^{31}$ représente un groupe physiologiquement acceptable, de caractère neutre ou basique, constitué de jusqu'à 3 α-aminoacides, dont la fonction carboxy terminale est libre, et présentant un point isoélectrique supérieur à 5,8, qui est caractérisé en ce que l'on fait réagir une insuline de formule I, dans laquelle

$R^1$ représente H ou H-Phe,

$R^{30}$ repésente le reste d'un L-aminoacide génétiquement codable et

$R^{31}$ représente OH ou un groupe protecteur de la fonction carboxy, avec un peptide de formule III

$$H—R^{30}—R^{31} \qquad (III),$$

dans laquelle

a) $R^{30}$ représente le reste d'un L-aminoacide neutre existant dans la nature, et

$R^{31}$ représente un groupe organique physiologiquement acceptable, de caractère basique, constitué de jusqu'à 3 α-aminoacides, dont la fonction carboxy terminale est libre ou,

b) $R^{30}$ représente le reste d'un L-aminoacide basique, existant dans la nature, et

$R^{31}$ représente un groupe organique physiologiquement acceptable, de caractère neutre ou basique, constitué de jusqu'à 3 α-aminoacides, dont la fonction carboxy terminale est libre, et présence d'une endopeptidase similaire à la trypsine, dans de l'eau, éventuellement avec addition d'un solvant organique approprié, à pH inférieur au point isoélectrique de l'insuline et éventuellement on élimine des groupes protecteurs introduits dans les chaîes latérales.

2. Procédé selon la revendication 1, caractérisé en ce que l'on prépare de dérivés d'insuline de formule I dans lesquels $R^1$ représente H-Phe.

3. Procédé selon l'une des revendications 1 ou 2, caractérisé en ce que l'on prépare des dérivés d'insuline de formule I dans lesquels, en tant que reste, $R^{30}$ représente un L-aminoacide neutre existant dans la nature Ala, Thr ou Ser.

4. Procédé selon une ou plusieurs des revendications 1 à 3, caractérisé en ce que l'on prépare des dérivés d'insuline de formule I dans lesquels la chaîne A et la chaîne (B2—30) comportent la séquence de l'insuline humaine.

5. Procédé selon une ou plusieurs des revendications 1 à 4, caractérisé en ce que l'on prépare l'insuline humaine-Arg$^{B31}$-Arg$^{B32}$-OH.

**Claims**

1. A process for the preparation of an insulin derivative of the formula I,

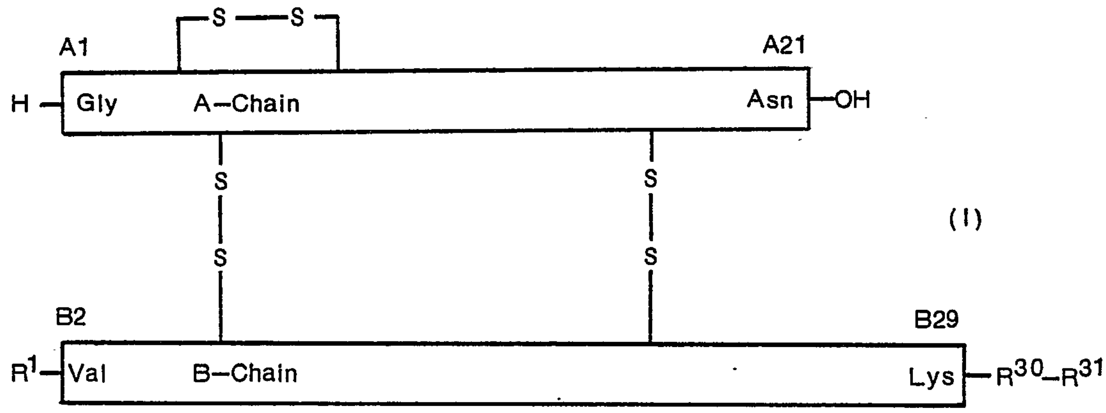

(I)

in which

$R^1$ denotes H or H-Phe,

a) $R^{30}$ represents the radical of a neutral, naturally occurring L-aminoacid and

$R^{31}$ represents a physiologically acceptable organic group of basic character, comprising up to 3 α-aminoacids in which the terminal carboxyl function is in the free form, or

b) $R^{30}$ represents the radical of a basic, naturally occurring L-aminoacid and

$R^{31}$ represents a physiologically acceptable organic group of neutral or basic character, comprising up to 3 α-aminoacids in which the terminal carboxyl function is in the free form and which insuline derivative has an isoelectric point above 5.8, which comprises reacting an insulin of the formula I in which

$R^1$ denotes H or H-Phe,

$R^{30}$ denotes the radical of a genetically codable L-aminoacid and

$R^{31}$ represents OH or a protective group of the carboxy function, with a peptide of the formula III in which

a) $R^{30}$ represents the radical of a neutral, naturally occurring L-aminoacid and

$R^{31}$ represents a physiologically acceptable organic group of basic character, comprising up to 3 α-aminoacids in which the terminal carboxyl function is in the free form, or

b) $R^{30}$ represents the radical of a basic, naturally occurring L-aminoacid and

$R^{31}$ represents a physiologicIly acceptable organic group of neutral or basic character, comprising up to 3 α-aminoacids in which the terminal carboxyl function is in the free form, in the presence of trypsin-like endopeptidase in water, if appropriate with the addition or a suitable organic solvent at a pH value below the isoelectric point of the insulin, and splitting off any protective groups introduced into the side chains.

2. The process as claimed in claim 1, wherein an insulin derivative of the formula I in which $R^1$ represents H-Phe is prepared.

3. The process as claimed in either of claims 1 or 2, wherein an insulin derivative of the formula I in which the radical $R^{30}$ represents a neutral, naturally occurring L-aminoacid from the group Ala, Thr or Ser is prepared.

4. The process as claimed in one or more of claims 1 to 3, wherein an insulin derivative of the formula I in which the A chain and the chain (B2—30) have the sequence of human insulin is prepared.

5. The process as claimed in one or more of claims 1 to 4, wherein human insulin-$Arg^{B31}$-OH or human insulin-$Arg^{B31}$-$Arg^{B32}$-OH is prepared.